# EUROPÄISCHE PATENTANMELDUNG

(11) **EP 3 348 283 A1**
(43) Veröffentlichungstag der Anmeldung: **18.07.2018**
(21) Anmeldenummer: 17151330.2
(22) Anmeldetag: 13.01.2017
(51) Int. Cl.: A61L 2/00, A61L 2/18, B08B 3/08, C11D 3/386, C11D 7/32

(54) **GROSSRAUM-DEKONTAMINATIONSANLAGE**

(71) Anmelder: Chemische Fabrik Dr. Weigert GmbH & Co. KG, 20539 Hamburg (DE)
(72) Erfinder: Staffeldt, Jürgen, 21423 Winsen (DE)
(74) Vertreter: Glawe, Delfs, Moll

(57) **Zusammenfassung**

Gegenstand der Erfindung ist eine Großraum-Dekontaminationsanlage, mit wenigstens einem Umwälztank mit einem Fassungsvermögen von wenigstens 60 1 Anwendungslösung. Erfindungsgemäß ist vorgesehen, dass sie eine Einrichtung zur separaten Dosierung von wenigstens zwei flüssigen Reinigerkomponenten (2, 3) im Reinigungsschritt aufweist.

## Beschreibung

Die Erfindung betrifft eine Großraum-Dekontaminationsanlage, mit wenigstens einem Umwälztank mit einem Fassungsvermögen von wenigstens 60 l Anwendungslösung. Gegenstand der Erfindung ist ferner eine Aufbereitungseinheit für Medizinprodukte (AEMP), sowie ein Verfahren zum Reinigen verschiedener im Krankenhausbereich eingesetzter Gegenstände.

Chirurgische Instrumente sowie andere medizinische Geräte werden im Krankenhaus üblicherweise unter Verwendung alkalischer Reinigungsmittel maschinell in einem Reinigung- und Desinfektionsgerät (RDG) gereinigt und anschließend chemisch oder thermisch desinfiziert. Ebenfalls bekannt ist es (WO 2015/043777 A1), bei der Reinigung einen enzymatischen, mildalkalischen Reiniger einzusetzen, der bedarfsweise aus zwei Komponenten hergestellt wird.

Größere Gegenstände aus dem Krankenhausbereich wie beispielsweise Transportwagen, OP-Tische und dergleichen werden regelmäßig in sogenannten Großraum-Dekontaminationsanlagen dekontaminiert, d. h. gereinigt und desinfiziert. Solche Dekontaminationsanlagen werden häufig mit RDG räumlich in einer sogenannten Aufbereitungseinheit für Medizinprodukte (AEMP) zusammengefasst.

Der Erfindung liegt die Aufgabe zugrunde, Vorrichtungen und Verfahren der eingangs genannten Art zu schaffen, die eine kostengünstige, einfache und wirkungsvolle Aufbereitung verschiedener Medizinprodukte ermöglichen.

Gegenstand der Erfindung ist eine Großraum-Dekontaminationsanlage, mit wenigstens einem Umwälztank mit einem Fassungsvermögen von wenigstens 60 l Anwendungslösung, dadurch gekennzeichnet, dass sie eine Einrichtung zur separaten Dosierung von wenigstens zwei flüssigen Reinigerkomponenten im Reinigungsschritt aufweist.

Zunächst seien einige im Rahmen der Erfindung verwendete Begriffe erläutert.

Eine erfindungsgemäße Großraum-Dekontaminationsanlage ist grundsätzlich ein Reinigungs-Desinfektionsgerät (RDG) gemäß Begriffsdefinition 3.62 der DIN EN ISO 15883-1:2006. Es dient somit der Reinigung und Desinfektion von Medizinprodukten und sonstigen im medizinischen, zahnmedizinischen, pharmazeutischen und veterinärmedizinischen Bereich verwendeten Gegenständen.

Der Begriff Großraum-Dekontaminationsanlage bezeichnet Reinigungs-Desinfektionsgeräte, die zur Reinigung größerer Medizinprodukte als übliche chirurgische und medizinische Instrumente vorgesehen sind. Insbesondere handelt es sich dabei um Reinigungs-Desinfektionsgeräte für nicht invasive, nicht kritische thermolabile Medizinprodukte und Zubehör im Gesundheitswesen gemäß DIN EN ISO 15883-7:2016. Darunter fallen beispielsweise Anlagen für die Aufbereitung von Bettgestellen, Nachttischen, Transportwagen, Containern, OP-Tischen, Sterilisationsbehältern, OP-Schuhen, Rollstühlen sowie Hilfsmitteln für Behinderte. Im allgemeinen Sprachgebrauch werden solche Anlagen beispielsweise als CWA (Containerwaschanlage) oder BWA (Bettgestell- und Wagendekontaminationsanlage) bezeichnet.

Im Umwälztank wird die Anwendungslösung (Wasser und zudosierte Inhaltsstoffe wie beispielsweise Reiniger), die bei Dekontaminationsverfahren im Umwälzsystem im Kreislauf geführt wird, vorbereitet und zwischengespeichert, darunter ist auch ein kombiniertes Frischwasser-Umwälzsystem zu verstehen, bei welchem z.B. nur die Dekontaminationsmittellösung umgewälzt wird. Der Begriff Fassungsvermögen bezeichnet die im Normalbetrieb vorgesehene Menge an Anwendungslösung in diesem Umwälztank.

Erfindungsgemäß ist vorgesehen, dass eine Einrichtung zur separaten Dosierung von wenigstens zwei flüssigen Reinigerkomponenten im Reinigungsschritt vorhanden ist. Die erfindungsgemäße Großraum-Dekontaminationsanlage wird daher mit einem Komponenten-System-Reiniger versehen, beispielsweise einem grundsätzlich aus WO 2015/043777 A1 bereits bekannten Komponentensystem. Die Erfindung hat erkannt, dass sich ein solches Zweikomponentensystem auch für Großraumanlagen nutzen lässt und dass ein besonderer Vorteil darin besteht, dass sich mit einem Komponentensystem durch entsprechende Einstellung von Menge und Verhältnis der Komponenten Reinigungsanforderungen sowohl für herkömmliche chirurgische Instrumente als auch für in entsprechenden Großraumanlagen zu dekontaminierende Gegenstände einstellen lassen. Somit ist es erfindungsgemäß möglich, eine Mehrzahl von oder alle in einem Krankenhaus anfallenden maschinellen Reinigungsaufgaben mit einem einzigen Komponentensystem eines Reinigers durchzuführen.

Eine erfindungsgemäße Großraum-Dekontaminationsanlage ist beispielsweise ausgewählt aus der Gruppe bestehend aus Container-Waschanlagen (CWA), Bettgestell- und Wagendekontaminationsanlagen (BWA), und Dekontaminationsanlagen für Transportbehälter, Nachttische, OP-Tische, Sterilisationsbehälter, OP-Schuhe, Rollstühle und Hilfsmittel für Behinderte. Es handelt sich hier um übliche, im Krankenhausbereich regelmäßig zu dekontaminierende Gegenstände.

Der wenigstens eine Umwälztank weist bevorzugt ein Fassungsvermögen von 60 bis 250 l, weiter vorzugsweise 60 bis 165 l, weiter vorzugsweise 80 bis 120 l Anwendungslösung auf. Wie unten noch darzulegen sein wird, kann auch bei solchen verhältnismäßig großen Tankvolumina mittels eines erfindungsgemäß verwendeten Komponentensystems eine schnelle und präzise Dosierung der erforderlichen Reinigermengen erfolgen.

Erfindungsgemäß kann vorgesehen sein, dass die Großraum-Dekontaminationsanlage eine Dosierstation mit wenigstens zwei Stellplätzen für Behälter mit Reinigungskomponenten mit einem Fassungsvermögen von 5 bis 50 l, vorzugsweise 5 bis 20 l, weiter vorzugsweise 5 bis 10 l, aufweist. Die Ausbildung des Reinigers als Komponentensystem erlaubt es, hochdosierte Konzentrate einzusetzen und gegebenenfalls in dieser Konzentration nicht kompatible Inhaltsstoffe in getrennten Komponenten zu dosieren, so dass sie erst verdünnt im Tank miteinander in Berührung kommen. Damit ist es möglich, auch bei erfindungsgemäßen Großraumanlagen Vorratsbehälter für Reinigerkomponenten mit verhältnismäßig geringen Volumina, beispielsweise höchstens 50 l oder höchstens 20 l, einzusetzen und mit diesem geringen Volumina eine ausreichende Betriebsdauer bis zum erforderlichen Wechsel des Behälters zu erreichen. Dies ist ein großer handhabungstechnischer Vorteil, da Großraumanlagen des Standes der Technik üblicherweise mit Reinigern aus Großgebinden gespeist werden müssen. Es ist erfindungsgemäß nicht erforderlich, für eine hinreichende Betriebsdauer große Volumina beispielsweise in Form von Fässern mit 200 l Inhalt zu bevorraten, die in der Regel ein aufwändiges System von Versorgungsleitungen benötigen, da Fässer mit einem solchen Inhalt üblicherweise nicht unmittelbar am Reinigungs- und Desinfektionsgerät gelagert werden können, sondern in einem separaten Lagerraum angeordnet werden und dementsprechend über lange Leitungen mit der Verbrauchsstelle verbunden werden müssen.

Aufgrund der erfindungsgemäß erforderlichen geringen Volumina von Reinigerkomponenten ist es möglich, dass die Dosierstation innerhalb des Bauraums bzw. Aggregateraums der Großraum-Dekontaminationsanlage angeordnet ist. Der Bauraum ist der von der Außenhülle der Anlage umschlossene Raum. Darin findet sich regelmäßig ein geeigneter Leerraum für die Unterbringung der verhältnismäßig kleinen Komponentengebinde der Erfindung.

Die Dosierpumpen weisen bevorzugt eine Förderleistung von 20 bis 60 ml/min auf. Dieser Bereich von Förderleistungen ermöglicht ein schnelles und präzises Dosieren der Komponenten.

Ein wesentlicher Vorteil des im Rahmen der Erfindung verwendeten Komponentensystems ist es, dass durch Anpassung der Mengen und Verhältnissen der Komponenten zueinander ein weiter Bereich unterschiedlicher Medizinprodukte mit demselben Komponentensystem gereinigt werden kann, ohne dass jeweils Spezialreiniger erforderlich wären. Erfindungsgemäß kann dabei vorgesehen sein, dass die Anlage wenigstens einen Sensor zum Erkennen des Typs der zu reinigenden Gegenstände und eine Einrichtung zur Auswahl eines geeigneten Reinigungs- und Desinfektionsverfahrens abhängig von diesem Typ aufweist, wobei diese Auswahl die Dosierung unterschiedlicher Mengen und/oder Mengenverhältnisse der beiden Komponenten im Reinigungsschritt umfasst. Der Sensor kann zum optischen Erkennen oder beispielsweise Abtasten der zu reinigenden Medizinprodukte ausgebildet sein, bevorzugt sind jedoch an den Medizinprodukten oder gegebenenfalls an deren Beladungsträgern mechanische und elektronische Codiereinrichtungen vorhanden, die mit entsprechenden Leseeinrichtungen der Anlage zusammenwirken. Beispielsweise kann es sich hier um RFID-Tags und RFID-Leseeinrichtungen handeln.

Ein weiterer Gegenstand der Erfindung ist eine Aufbereitungseinheit für Medizinprodukte (AEMP), mit wenigstens einer erfindungsgemäßen Großraum-Dekontaminationsanlage und wenigstens einem Reinigungs- und Desinfektionsgerät (RDG) mit wenigstens einem Umwälztank mit einem Fassungsvermögen von höchstens 50 l Anwendungslösung, dadurch gekennzeichnet, dass die AEMP wenigstens eine Einrichtung zur separaten Dosierung von wenigstens zwei flüssigen Reinigerkomponenten im Reinigungsschritt aufweist, die zur Zufuhr der gleichen Reinigerkomponenten zu der wenigstens einen Großraum-Dekontaminationsanlage und dem wenigstens einen Reinigungs- und Desinfektionsgerät ausgebildet ist. Die gesamte AEMP erfordert somit nur die Bevorratung eines einzigen Reinigersystems für alle darin anfallenden Reinigungsaufgaben. Erfindungsgemäß müssen somit weder große Behälter noch eine Vielzahl verschiedener Reiniger für die unterschiedlichen Reinigungsaufgaben vorgehalten werden.

Es ist im Rahmen der Erfindung möglich, alle RDG bzw. Dekontaminationsanlagen einer AEMP zentral von einer einzigen Dosierstation aus zu speisen. Häufig ist es jedoch bevorzugt, zwei oder mehr dezentrale Dosierstationen vorzusehen, die jeweils mit identischen Reinigerkomponenten versehen sind. Dementsprechend ist bei einer bevorzugten Ausführungsform der Erfindung vorgesehen, dass sie zwei oder mehr Dosierstationen mit jeweils wenigstens zwei Stellplätzen für Behälter mit Reinigungs/Desinfektionskomponenten mit einem Fassungsvermögen von 5 bis 50 l, vorzugsweise 5 bis 20 l, weiter vorzugsweise 5 bis 10 l, aufweist.

Ein weiterer Gegenstand der Erfindung ist die Verwendung von zwei flüssigen Reinigerkomponenten, die beinhalten:
a) eine erste Komponente, die wenigstens 15 Gew.-% Alkanolamin und wenigstens 10 Gew.-% Komplexbildner enthält;
b) eine zweite Komponente, die wenigstens ein Enzym enthält;
zur separaten Dosierung im Reinigungsschritt einer erfindungsgemäßen Großraum-Dekontaminationsanlage oder Aufbereitungseinheit für Medizinprodukte.

Die erste Komponente eines solchen Kits (auch Alkalikomponente genannt) enthält verhältnismäßig hohe Konzentrationen Alkanolamin und Komplexbildner. Die zweite Komponente (auch Enzymkomponente genannt) enthält wenigstens ein Enzym, vorzugsweise ein proteolytisches Enzym. Die Menge dieser proteolytischen Enzyme liegt bevorzugt zwischen 0,05 und 1 Anson-Einheiten pro g Komponente.

Überraschenderweise hat sich gezeigt, dass sich durch Aufteilen dieser grundsätzlich bekannten Bestandteile eines Reinigungsmittels auf zwei Komponenten in der beanspruchten Art und Weise eine sehr wirksame Reinigung mit sehr geringen Wirkstoffmengen und vor allen Dingen auch mit sehr geringen eingesetzten Volumina der beiden Komponenten erreichen lässt.

Es hat sich gezeigt, dass der Einsatz eines Alkanolamins als Bestandteil der alkalischen Komponente in der beanspruchten Mindestkonzentration zu einer besonders guten Wirksamkeit bei sehr geringen Dosiermengen bzw. Konzentrationen der Komponenten des Kits in die wässrige Anwendungslösung führen. Die erfindungsgemäß vorgesehene Auftrennung der Bestandteile des Reinigungsmittels auf zwei Komponenten erlaubt das Bereitstellen so genannter Hochkonzentrate, bei denen nicht oder nur schwer miteinander kompatible Inhaltsstoffe (beispielsweise alkalische Alkanolamine einerseits und Enzym andererseits) in höheren Konzentrationen stabil gelagert und vorrätig gehalten werden können als bei einem Reinigungsmittel mit lediglich einer Komponente.

Der Alkanolamingehalt der ersten Komponente beträgt bei einer bevorzugten Ausführungsform der Erfindung 15-30 Gew.-%, weiter vorzugweise 15-25 Gew.-%. Das Alkanolamin kann bevorzugt die folgende Struktur aufweisen: 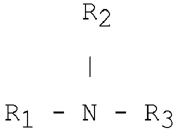 wobei R₁ eine Hydroxyalkylgruppe mit 1 bis 6 C-Atomen darstellt und wobei R₂ und R₃ unabhängig voneinander die genannte Hydroxyalkylgruppe oder Wasserstoff darstellen.

Weiter bevorzugt ist das Alkanolamin ausgewählt aus der Gruppe bestehend aus Mono-, Di- und/oder Triethanolamin.

Die erste Komponente kann bevorzugt 10-30 Gew.-%, weiter vorzugsweise 15-25 Gew.-% Komplexbildner enthalten. Die Komplexbildner dienen der Wasserenthärtung und können durch Komplexieren von Erdalkalionen die Reinigungswirkung gegenüber OP-typischen Verunreinigungen verbessern. Bei den Komplexbildnern kann es sich um Homo-, Co- oder Terpolymere auf der Basis von Acrylsäure oder deren Alkalisalzen handeln, ferner um Phosphonsäuren bzw. deren Alkalisalze, wie bspw. 1-Hydroxyethan-1,1-diphosphonsäure, Aminotrismethylenphosphonsäure, Ethylendiaminotetrakismethylenphosphonsäure, Phosphonobutantricarbonsäure; Weinsäure, Citronensäure und Glukonsäure; ferner Nitrilotriessigsäure oder Ethylendiaminotetraessigsäure bzw. deren Salze. Bevorzugt ist die Verwendung von Chelatbildnern, insbesondere Amino(poly)carbonsäuren sowie deren Salzen, beispielsweise das Trinatriumsalz der Methylglycindiessigsäure (MGDA).

Die korrosive Wirkung von Komplexbildnern auf eloxierte Aluminiumoberflächen und dergleichen kann vermindert bzw. vollständig vermieden werden durch den Zusatz wenigstens eines Mono- und/oder Diesters der Phosphorsäure mit aliphatischen Alkolholen der Kettenlänge C₁ bis C₂₂ und/oder aliphatischen Diolen und/oder aliphatischen Polyolen der Kettenlänge C₂ bis C₂₂. Besonders bevorzugt ist ein Diester der Phosphorsäure mit Butanol einerseits und Ethylenglykol andererseits. Dieser Ester ist kommerziell unter der Bezeichnung Hordaphos® MDGB erhältlich. Erfindungsgemäß erhält man so eine gute Reinigungswirkung auch bei Verwendung harten Wassers und trotzdem eine schonende Einwirkung auf eloxierte Aluminiumflächen.

Die zweite Komponente kann wenigstens ein proteolytisches Enzym enthalten. Geeignete proteolytische Enzyme sind bspw. von der Fa. Novozymes unter der Bezeichnung Esperase® 8.0 L, Subtilisin®, Liquanase® Ultra 2.0 L oder Liquanase Eviity 2.0 XL erhältlich.

Die zweite Komponente kann erfindungsgemäß Tenside, vorzugsweise nichtionische Tenside, enthalten. Der Zusatz anionischer und kationischer Tenside ist ebenfalls möglich.

Die nichtionischen Tenside können erfindungsgemäß ausgewählt sein aus der Gruppe bestehend aus Fettalkoholethoxylaten, Fettalkoholpropoxylaten, EO-PO-Blockcopolymeren, Alkylglucosiden, Alkylpolyglucosiden, Oktylphenolethoxylaten und Nonylphenolethoxylaten. Dem Fachmann geläufige ethoxylierte und/oder propoxylierte Fettalkohole sind besonders bevorzugt. Solche nichtionischen Tenside sind beispielsweise von den Firmen Cognis oder Julius Hoesch erhältlich.

Insbesondere der zweiten Komponente können übliche Konservierungsmittel zugesetzt werden, bspw. p-Hydroxybenzoesäure oder deren Methylester, 5-Brom-5-Nitro-1,3-dioxan, Salicylsäure, 2-Naphtyl-m-N-Dimethylthiocarbanilat, 5-Chlor-5-methyl-4-isothiazolin-3-on, 2-Methyl-4-isothiazolin-3-on, Cetyltrimethylammoniumchlorid (CTAC) sowie Gemische dieser Verbindungen. Ein weiteres Konservierungsmittel ist p-Hydroxybenzoesäure bzw. deren Methylester. Mit Hilfe dieser Konservierungsmittel läßt sich Mikroben- und Pilzbefall der Komponenten vermeiden.

Bei Bedarf können Konfektionierhilfsmittel (Lösungsvermittler) zugegeben werden wie bspw. Natriumcumolsulfonat, Natriumtoluolsulfonat, Natriumxylolsulfonat, Harnstoff, Glykole, insbesondere Polypropylenglykole und Polyethylenglykole, Methylacetamid und Fettalkohole, wie bzw. Cetylalkohol.

Die Aufzählung möglicher Inhaltsstoffe ist nicht abschließend. Es können zusätzlich bspw. Netzmittel, Emulgatoren, schaumbremsende Mittel oder dergleichen zugesetzt werden. Vorteilhaft ist beispielsweise der Zusatz von N-Acylglutamat als Netzmittel.

Die wässrige Lösung der Komponenten des Kits weist bevorzugt einen pH-Wert von 9 bis 11, weiter vorzugsweise 10 bis 11 auf. Sofern im Rahmen der vorliegenden Anmeldung pH-Werte einer verdünnten Lösung des Reinigungsmittelkonzentrats gemessen werden, wird als Lösungsmittel vollentsalztes Wasser (VE-Wasser) verwendet. Wenn das Konzentrat mit üblichem Leitungswasser zu einer gebrauchsfertigen Lösung angesetzt wird, können sich je nach Beschaffenheit dieses Wassers geringfügig abweichende pH-Werte ergeben.

Die Einstellung des pH-Werts der Konzentrate/Komponenten auf den gewünschten Bereich erfolgt ggf. vorzugsweise durch Zusatz von Säuren und/oder geeigneter Puffersysteme. Bevorzugt ist der Zusatz von wenigstens einer organischen Säure ausgewählt aus der Gruppe bestehend aus Mono-, Di- oder Tricarbonsäuren mit 2 bis 6 C-Atomen. Unter diesen Säuren sind bevorzugt Citronensäure, Weinsäure, Apfelsäure, Milchsäure, Glykolsäure, Glyoxylsäure, Bernsteinsäure, Adipinsäure und Glutarsäure. Citronensäure ist besonders bevorzugt. Die Säuren werden dem Konzentrat vorzugsweise in geringen Mengen zugesetzt. Einige dieser genannten organischen Säuren wie beispielsweise Citronensäure sind gleichzeitig Komplexbildner im Sinne der Erfindung.

Bevorzugt ist es, wenn die Dosierung unterschiedlicher Mengen und/oder Mengenverhältnisse der beiden Komponenten im Reinigungsschritt in Abhängigkeit vom Typ der zu reinigenden Gegenstände erfolgt. Dabei kann bevorzugt eine automatische Erkennung des Typs der zu reinigenden Gegenstände mittels wenigstens eines Sensors erfolgen, wie oben im Kontext der Großraum-Dekontaminationsanlage bereits beschrieben.

Ein weiterer Gegenstand der Erfindung ist ein Verfahren zum Reinigen von Gegenständen ausgewählt aus der Gruppe bestehend aus Bettgestellen, Nachttischen, Transportbehältern, Transportwagen, Containern, OP-Tischen, Sterilisationsbehältern, Rollstühlen, und Hilfsmitteln für Behinderte, in einer erfindungsgemäßen Großraum-Dekontaminationsanlage, gekennzeichnet durch folgende Schritte:
a) Aufbringen einer 0,02 bis 0,3%igen wässrigen Anwendungslösung wenigstens einer der beiden folgenden Komponenten:
   aa) eine erste Komponente, die wenigstens 15 Gew.-% Alkanolamin und wenigstens 10 Gew.-% Komplexbildner enthält;
   bb) eine zweite Komponente, die wenigstens ein Enzym enthält;
b) Einwirkenlassen der Lösung bei einer Temperatur von Raumtemperatur bis zur Siedetemperatur der Lösung,
c) Nachspülen.

Das Einwirkenlassen kann nach dem Aufbringen der Anwendungslösung ein Einwirken in Ruhe, d.h. ohne fortlaufendes Aufbringen bzw. Aufsprühen oder mechanisches Umwälzen bzw. Bewegen der Anwendungslösung umfassen. Bevorzugt ist im Zuge des maschinellen Reinigens ein ständiges oder unterbrochenes Umwälzen der Anwendungslösung, dabei wird die Lösung bevorzugt fortlaufend auf die Gegenstände aufgesprüht bzw. aufgespült.

Die Komponenten können unmittelbar in die als Anwendungslösung verwendete wässrige Lösung eindosiert werden.

Das Einwirkenlassen in Schritt b) erfolgt bevorzugt bei Raumtemperatur bis 55°C, vorzugsweise bei 35 bis 50°C, weiter vorzugsweise bei 40 bis 50°C.

Erfindungsgemäß ist bevorzugt, dass die Dosiermengen jeder Komponente in einem Reinigungsgang 500 ml oder weniger, vorzugsweise 200 ml oder weniger, weiter vorzugsweise 165 ml oder weniger betragen. Das Dosieren solcher geringen Mengen auch in Großraum-Dekontaminationsanlage mit entsprechend großen Tanks ist durch den erfindungsgemäßen Einsatz der genannten Konzentrate möglich und erlaubt ein sehr schnelles Einstellen der gewünschten Konzentration in der Anwendungslösung und damit eine kürzere Zyklenzeit.

Erfindungsgemäß kann eine automatische Erkennung des Typs der zu reinigenden Gegenstände mittels wenigstens eines Sensors und eine Dosierung unterschiedlicher Mengen und/oder Mengenverhältnisse der beiden Komponenten im Reinigungsschritt in Abhängigkeit vom Typ der zu reinigenden Gegenstände erfolgen, wie vorstehend im Kontext der erfindungsgemäßen Vorrichtung beschrieben.

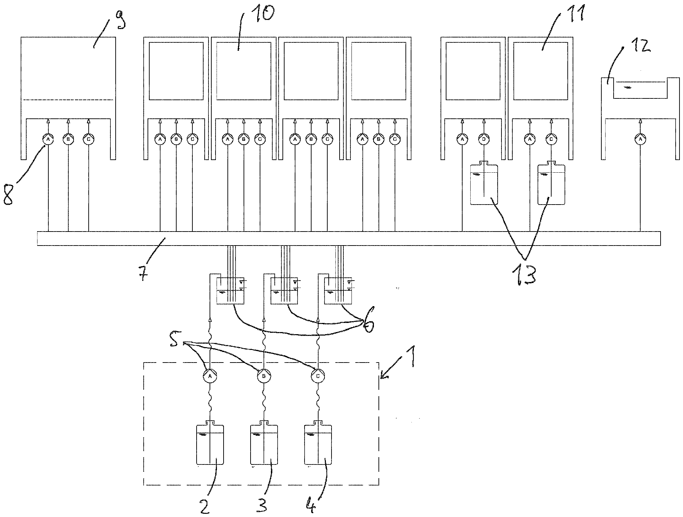

Ausführungsbeispiele der Erfindung werden nachfolgend beschrieben. Die Figur zeigt schematisch eine erfindungsgemäße AEMP.

### Beispiel 1

Eine erfindungsgemäße Alkalikomponente wird anhand der Angaben der nachfolgenden Tabelle zubereitet. Die Mengen der einzusetzenden Ausgangsstoffe sind in Gewichtsteilen angegeben.

| | |
|---|---|
| Triethanolamin 99% | 20,0 |
| MDGB¹ | 2,0 |
| KOH, 45% | 3,0 |
| MGDA 3 Na, 40% | 40,0 |
| Acumer^{®} 2000² | 3,5 |
| Wasser (vollentsalzt) | 31,5 |
| pH-Wert des Konzentrats | 11,8 |
| pH-Wert einer 0,1%igen wässrigen Lösung (in VE-Wasser) | 10,8 |

| | |
|---|---|
| ¹ Diester der Phosphorsäure mit Butanol und Ethylenglykol ² Komplexbildner auf Basis von Carboxylat/Sulfonat-Acrylcopolymeren, Fa. Rohm and Haas | |

Eine erfindungsgemäße Enzymkomponente wird wie folgt hergestellt:

| | |
|---|---|
| Na-Cumolsulfonat 40% | 5,0 |
| Subtilisin | 10,0 |
| FA C12/C14; 2EO/4PO³ | 0,8 |
| FA C12/C14; 5EO/4PO | 3,0 |
| FA C12/C15; 2EO/6PO | 0,1 |
| CTAC | 0,2 |
| Mischung Zitr./Apfelsäure 40% | 0,28 |
| KOH, 45% | 0,02 |
| Wasser (vollentsalzt) | 80,6 |
| pH-Wert des Konzentrats | 7,2 |
| pH-Wert einer 0,1%igen wässrigen Lösung (in VE-Wasser) | 7,1 |

| | |
|---|---|
| ³ Ethoxylierte/propoxylierte Fettalkohole | |

### Beispiel 2

In diesem Beispiel werden Programme zur Reinigung verschiedener Medizinprodukte unter Verwendung der Komponenten des Beispiels 1 genannt.

### Programme für Großraum-Dekontaminationsanlage

### Programm für eloxierte Aluminiumcontainer und für eloxierte Aluminium-Transportwagen

Für beide vorgenannten Aufbereitungsgüter kann aus Gründen des Materialschutzes hauptsächlich die pH-neutrale Enzymkomponente dosiert werden und die Alkalikomponente im Verhältnis dazu gering dosiert werden.

| | |
|---|---|
| Programmablauf: | - Vorspülung mit Kaltwasser 1 min |
| | - Reinigung mit 0,08 % Enzymkomponente und 0,05 % Alkalikomponente 5 min bei 45 °C |
| | - Nachspülung/Thermodesinfektion mit Wasser bei 90 °C 1 min |

### Programm für OP-Schuhe

OP-Schuhe, üblicherweise aus Polyurethan, sind weniger empfindlich gegenüber Alkalien. Da sie aber blutbehaftet sein können bietet sich ein Programm an, welches beide Komponenten in gleicher Konzentration berücksichtigt.

| | |
|---|---|
| Programmablauf: | - Vorspülung mit Kaltwasser 1 min |
| | - Reinigung mit 0,08 % Enzymkomponente und 0,08 % Alkalikomponente 5 min bei 45 °C |
| | - Nachspülung/Thermodesinfektion mit Wasser bei 75 °C 2 min |

### Einsatz im Ultraschallbecken

Das Ultraschallbecken dient der manuellen Vorreinigung stark verschmutzter Instrumente. Es werden enzymatische Reiniger eingesetzt. Hierfür bietet sich die Enzymkomponente an, Konzentration 0,1 %.

| | |
|---|---|
| Ultraschallreinigung: | |
| | - Reinigung mit 0,1 % Enzymkomponente 5 min bei 20 bis 40 °C |

### Programme für Instrumenten-RDG

### Programm für chirurgische Instrumente

An chirurgischen Instrumenten haftet nicht nur Blut in verschiedenen Antrocknungsstufen sondern auch Gewebe, Fett, Hautdesinfektionsmittel, Koagulationsrückstände etc. Aus diesem Grunde bietet sich eine Kombination mit Enzymen/Tensiden und erhöhter Alkalität an.

| | |
|---|---|
| Programmablauf: | - Vorspülung mit Kaltwasser 1 min |
| | - Reinigung mit 0,08 % Enzym/Tensidkomponente und 0,10 % Alkalikomponente 10 min bei 45 bis 55 °C |
| | - Zwischenspülung mit Wasser |
| | - Nachspülung/Thermodesinfektion mit Wasser bei 90 °C 5 min |

### Programm für Endoskope im RDG-E

Flexible Endoskope sollten aus Gründen des Materialschutzes im pH-neutralen Bereich gereinigt werden. Hier bietet sich die Enzymkomponente an.

| | |
|---|---|
| Programmablauf: | - Vorspülung mit Kaltwasser 3 min |
| | - Reinigung mit 0,05 % Enzym/Tensidkomponente 5 min bei 45 °C |
| | - Chemo-thermische Desinfektion bei 55 °C, 5 min mit neodisher® endoSept GA |
| | - Nachspülung mit Wasser |

### Beispiel 3

In der Figur ist schematisch der Aufbau einer erfindungsgemäßen Aufbereitungseinheit für Medizinprodukte dargestellt.

In einer Dosierzentrale 1 sind drei Vorratsbehälter für Konzentrat angeordnet. Es kann sich um Kanister mit einem Fassungsvermögen von 20 l handeln. Im Vorratsbehälter 2 befindet sich ein erfindungsgemäßes Enzymkonzentrat, im Vorratsbehälter 3 ein erfindungsgemäßes Alkalikonzentrat und im Vorratsbehälter 4 ein Klarspülerkonzentrat des Standes der Technik.

Jedem Vorratsbehälter ist eine Befüllpumpe 5 zugeordnet, mittels der der Inhalt der Vorratsbehälter in Vorlagebehälter 6 gepumpt werden kann. In dem Ausführungsbeispiel sind sämtliche Pumpen mit A, B oder C gekennzeichnet; die mit A gekennzeichneten Pumpen fördern das Enzymkonzentrat, die mit B gekennzeichneten Pumpen das Alkalikonzentrat und die mit C gekennzeichneten Pumpen das Klarspülerkonzentrat.

In die Vorlagebehälter 6 ragen Entnahmeleitungen eines insgesamt mit 7 bezeichneten Leitungssystems, durch das der Inhalt der Vorlagebehälter einer Mehrzahl von Dosierpumpen 8 zugeführt werden kann. Diese Dosierpumpen 8 dosieren die Komponenten in die entsprechenden Einrichtungen der erfindungsgemäßen AEMP.

Die Bezugsziffer 9 bezeichnet eine erfindungsgemäße Großraum-Dekontaminationsanlage, die Bezugsziffern 10 eine Anordnung von vier Reinigungs- und Desinfektionsgeräten (RDG). Diese Einrichtungen werden sämtlich bedarfsweise mit allen drei Komponenten aus den Vorlagebehältern 6 gespeist.

Die Bezugsziffer 11 bezeichnet eine Anordnung von zwei RDG für Endoskope. Diese werden aus dem Leitungssystem 7 lediglich mit der Enzymkomponente aus dem Vorratsbehälter 2 gespeist. Lokal im Bereich dieser RDG ist jeweils ein separater Vorratsbehälter 13 für eine Desinfektionskomponente angeordnet.

Das Ultraschallbecken 12 wird ausschließlich mit der Enzymkomponente aus dem Vorratsbehälter 2 gespeist.

## Patentansprüche

1. Großraum-Dekontaminationsanlage, mit wenigstens einem Umwälztank mit einem Fassungsvermögen von wenigstens 60 l Anwendungslösung, **dadurch gekennzeichnet, dass** sie eine Einrichtung zur separaten Dosierung von wenigstens zwei flüssigen Reinigerkomponenten (2, 3) im Reinigungsschritt aufweist.

2. Großraum-Dekontaminationsanlage, nach Anspruch 1, **dadurch gekennzeichnet, dass** sie ausgewählt ist aus der Gruppe bestehend aus Container-Waschanlagen (CWA), Bettgestell- und Wagendekontaminationsanlagen (BWA), und Dekontaminationsanlagen für Transportbehälter, Nachttische, OP-Tische, Sterilisationsbehälter, OP-Schuhe, Rollstühle und Hilfsmittel für Behinderte.

3. Großraum-Dekontaminationsanlage, nach Anspruch 1 oder 2, **dadurch gekennzeichnet, dass** der wenigstens eine Umwälztank ein Fassungsvermögen von 60 bis 250 l, vorzugsweise 60 bis 165 l, weiter vorzugsweise 80 bis 120 l Anwendungslösung aufweist.

4. Großraum-Dekontaminationsanlage nach einem der Ansprüche 1 bis 3, **dadurch gekennzeichnet, dass** sie eine Dosierstation (1) mit wenigstens zwei Stellplätzen für Behälter (2, 3) mit Reinigungs/Desinfektionskomponenten mit einem Fassungsvermögen von 5 bis 50 l, vorzugsweise 5 bis 20 l, weiter vorzugsweise 5 bis 10 l, aufweist.

5. Großraum-Dekontaminationsanlage, nach Anspruch 4, **dadurch gekennzeichnet, dass** die Dosierstation (1) innerhalb des Bauraums bzw. Aggregateraums der Großraum-Dekontaminationsanlage angeordnet ist.

6. Großraum-Dekontaminationsanlage, nach Anspruch 4 oder 5, **dadurch gekennzeichnet, dass** die Dosierpumpen (8) eine Förderleistung von 20 bis 60 ml/min aufweisen.

7. Großraum-Dekontaminationsanlage nach einem der Ansprüche 1 bis 6, **dadurch gekennzeichnet, dass** sie wenigstens einen Sensor zum Erkennen des Typs der zu reinigenden Gegenstände und eine Einrichtung zur Auswahl eines geeigneten Reinigungs- und Desinfektionsverfahrens abhängig von diesem Typ aufweist, wobei diese Auswahl die Dosierung unterschiedlicher Mengen und/oder Mengenverhältnisse der beiden Komponenten im Reinigungsschritt umfasst.

8. Aufbereitungseinheit für Medizinprodukte (AEMP), mit wenigstens einer Großraum-Dekontaminationsanlage (9) nach einem der Ansprüche 1 bis 7 und wenigstens einem Reinigungs- und Desinfektionsgerät (RDG) (10) mit wenigstens einem Umwälztank mit einem Fassungsvermögen von höchstens 50 l Anwendungslösung, **dadurch gekennzeichnet, dass** die AEMP wenigstens eine Einrichtung zur separaten Dosierung von wenigstens zwei flüssigen Reinigerkomponenten (2, 3) im Reinigungsschritt aufweist, die zur Zufuhr der gleichen Reinigerkomponenten zu der wenigstens einen Großraum-Dekontaminationsanlage und dem wenigstens einen Reinigungs- und Desinfektionsgerät ausgebildet ist.

9. Aufbereitungseinheit für Medizinprodukte nach Anspruch 8, **dadurch gekennzeichnet, dass** sie zwei oder mehr Dosierstationen mit jeweils wenigstens zwei Stellplätzen für Behälter mit Reinigungs/Desinfektionskomponenten mit einem Fassungsvermögen von 5 bis 50 l, vorzugsweise 5 bis 20 l, weiter vorzugsweise 5 bis 10 l aufweist.

10. Verwendung von zwei flüssigen Reinigerkomponenten, die beinhalten:
a) eine erste Komponente, die wenigstens 15 Gew.-% Alkanolamin und wenigstens 10 Gew.-% Komplexbildner enthält;
b) eine zweite Komponente, die wenigstens ein Enzym enthält;
zur separaten Dosierung im Reinigungsschritt einer Großraum-Dekontaminationsanlage nach einem der Ansprüche 1 bis 7 oder Aufbereitungseinheit für Medizinprodukte nach Anspruch 8 oder 9.

11. Verwendung nach Anspruch 10, **dadurch gekennzeichnet, dass** die Dosierung unterschiedlicher Mengen und/oder Mengenverhältnisse der beiden Komponenten im Reinigungsschritt in Abhängigkeit vom Typ der zu reinigenden Gegenstände erfolgt.

12. Verwendung nach Anspruch 11, **dadurch gekennzeichnet, dass** eine automatische Erkennung des Typs der zu reinigenden Gegenstände mittels wenigstens eines Sensors erfolgt.

13. Verfahren zum Reinigen von Gegenständen ausgewählt aus der Gruppe bestehend aus Bettgestellen, Nachttischen, Transportbehältern, Transportwagen, Containern, OP-Tischen, Sterilisationsbehältern, Rollstühlen, und Hilfsmitteln für Behinderte, in einer Großraum-Dekontaminationsanlage nach einem der Ansprüche 1 bis 7; **gekennzeichnet durch** folgende Schritte:
a) Aufbringen einer 0,02 bis 0,3%igen wässrigen Lösung wenigstens einer der beiden folgenden Komponenten:
aa) eine erste Komponente, die wenigstens 15 Gew.-% Alkanolamin und wenigstens 10 Gew.-% Komplexbildner enthält;
bb) eine zweite Komponente, die wenigstens ein Enzym enthält;
b) Einwirkenlassen der Lösung bei einer Temperatur von Raumtemperatur bis zur Siedetemperatur der Lösung,
c) Nachspülen.

14. Verfahren nach Anspruch 13, **dadurch gekennzeichnet, dass** die Dosiermengen jeder Komponente in einem Reinigungsgang 500 ml oder weniger, vorzugsweise 200 ml oder weniger, weiter vorzugsweise 165 ml oder weniger betragen.

15. Verfahren nach Anspruch 13 oder 14, **dadurch gekennzeichnet, dass** eine automatische Erkennung des Typs der zu reinigenden Gegenstände mittels wenigstens eines Sensors und eine Dosierung unterschiedlicher Mengen und/oder Mengenverhältnisse der beiden Komponenten im Reinigungsschritt in Abhängigkeit vom Typ der zu reinigenden Gegenstände erfolgt.
